# EUROPEAN PATENT APPLICATION

(11) **EP 3 636 172 A1**
(43) Date of publication of application: **15.04.2020**
(21) Application number: 18199980.6
(22) Date of filing: 11.10.2018
(51) Int. Cl.: A61B 17/12, A61B 17/34, A61B 18/14, A61B 18/02, A61B 17/00, A61B 18/00

(54) **A DEVICE FOR ACCESSING THE EPICARDIAL SPACE**

(71) Applicant: Aurigen Medical Limited, Galway H91T8NW (IE)
(72) Inventor: O'Halloran, Tony, Turloughmore, Co. Galway (IE); Thompson, John, Balgriffin, Dublin 13 (IE)
(74) Representative: Purdylucey Intellectual Property

(57) **Abstract**

A device (1) for providing transluminal access to the epicardial space (2) via the left atrial appendage (LAA) (3) comprises an implantable occlusion apparatus (4) configured for detachable attachment to an elongated catheter member and adjustment between a contracted orientation suitable for transluminal delivery and a deployed orientation configured to occlude the LAA. The implantable occlusion apparatus has a through-conduit (5) and a cover (6) disposed on a proximal end configured to fluidically isolate the LAA from the left atrium (7) when the occlusion apparatus is deployed within the LAA, and the cover comprises an opening (8) to the through-conduit and a closure (9) for the opening. A wall piercing arm (10, 20) configured to cooperate with the implantable occlusion apparatus is provided and configured to generate a hole (12) in a wall of the LAA distal of the occlusion apparatus during use to provide access to the epicardial space (2) via the LAA. The wall piercing arm comprises a distal wall piercing head selected from a wall-piercing needle (21), ultra-sonic probe, or tissue ablation electrode.

## Description

### Field of the Invention

The present invention relates to a device for accessing the epicardial space.

### Background to the Invention

The heart is surrounded by a membrane called the pericardium, and the space defined by the heart and pericardium is called the epicardial space. This space is difficult to access, and generally requires an apical approach through the ribcage and muscle. This procedure is risky, time-consuming and lengthy, even when performed by means of keyhole surgery, and requires the skills of a cardiothoracic surgeon.

It is an object of the invention to overcome at least one of the above-referenced problems.

### Summary of the Invention

The invention provides a device and method for percutaneous (transluminal) access to the epicardial space, that can be implanted in the LAA of the left atrium of the heart, fluidically isolating the left atrium from the LAA, and can generate a hole in the wall of the LAA providing access to the epicardial space through the LAA. The device comprises an implantable occlusion apparatus (for example a radially expansible cage) configured for transluminal delivery and then deployment in the mouth of the LAA to occlude the LAA. The proximal end of the device may have a cover which fluidically isolated the left atrium from the LAA when the device is deployed. The occlusion device has a through-conduit having a proximal opening, and a closure for the opening (generally part of the cover, for example a re-closable flap or polymeric leaflet-type valve). The through-conduit allows a catheter to be advanced through the occlusion device, and also allows modules to be accommodated within the device. The device comprises a piercing arm configured to pierce a hole in the wall of the LAA. The arm may be part of the occlusion apparatus (for example, the arm may be attached to a module disposed within the through conduit), or may be a separate catheter that is configured to pass through the through-conduit of the occlusion apparatus. Once a hole is pierced in the wall of the LAA, substances can be delivered into the epicardial space through the hole, and treatment or sensing apparatus can be advanced through the occlusion apparatus and into the epicardial space via the LAA.

In a first aspect, the invention provides device for providing transluminal access to the epicardial space via the left atrial appendage comprising:
an implantable occlusion apparatus configured for detachable attachment to an elongated catheter member and adjustment between a contracted orientation suitable for transluminal delivery and a deployed orientation configured to occlude the LAA, the implantable occlusion apparatus having a through-conduit and a cover disposed on a proximal end configured to fluidically isolate the LAA from the left atrium when the occlusion apparatus is deployed within the LAA, the cover comprising an opening to the through-conduit and a closure for the opening; and
a wall piercing arm operably configured to cooperate with the implantable occlusion apparatus and generate a hole in a wall of the LAA distal of the occlusion apparatus during use to provide access to the epicardial space via the left atrial appendage.

In one embodiment, the wall piercing arm comprises a distal wall piercing head selected from a wall-piercing needle, ultra-sonic probe, or tissue ablation electrode.

In one embodiment, the wall piercing arm is operatively coupled to the implantable occlusion apparatus. In one embodiment, the wall piercing arm is attached to a control module disposed within the through conduit. The control module is configured for detachable engagement in the through-conduit.

In one embodiment, the wall piercing arm comprises a substance delivery lumen configured to deliver a substance (for example a drug or a cooling fluid) into the epicardial space. In one embodiment, the lumen is provided in a wall piercing needle. A cooling may be applied to the epicardial space to reduce post-myocardial infarction inflammation.

In one embodiment, the control module comprises a reservoir for substance that is configured for fluidic connection to the lumen of the wall piercing arm.

In one embodiment, the device comprises a plurality of wall piercing arms. In one embodiment, the or each arm may comprise a lumen for transferring a substance from the occlusion apparatus to the epicardial space, or an energy delivery electrode.

In one embodiment, the wall piercing arm is axially adjustable relative to the implantable occlusion apparatus. The wall piercing arm may be adjustable from a delivery configuration (for example, constrained within the module or a delivery catheter) to a deployed configuration in which the end of the arm abuts a wall of the LAA (for example, angled outwardly). The arm may comprise a shape-memory material, for example nitinol.

In another embodiment, the wall piercing arm comprises an elongated wall piercing catheter configured for insertion through the through-conduit of the deployed radially expansible element, and (a) into the occluded LAA (transluminal approach) or (b) into the left atrium (apical approach).

In one embodiment, the wall piercing catheter is configured for guided movement.

In one embodiment, the wall piercing catheter has a distal end configured to treat or stimulate tissue, or sense a parameter of tissue.

The cover on the occlusion apparatus may be integrally formed with the occlusion apparatus, or may be a separate cover applied to the proximal end of the occlusion apparatus. The cover may be a mesh. The cover generally has an opening that overlies the opening into the through-conduit, and an associated closure for the opening. The opening may be a flap of material, or may be valve (such as a polymer leaflet-type valve). The closure is preferably self-closing.

The occlusion apparatus may be a radially expansible cage, and may be formed from wires, for example stainless steel or nitinol wires. One or more of the wires may comprise a brush member comprising a multiplicity of bristles. The bristles may be porous. In one embodiment, the occlusion apparatus is generally cylindrical, and may have a toroidal proximal end and may have a cylindrical distal end. The through conduit generally extends through a central axis of the cage. The through conduit may be expandable.

The invention also relates to a method of for providing transluminal access to the epicardial space via the left atrial appendage employing a device of the invention and comprising the steps of:
deploying an occlusion apparatus of the device of the invention in the LAAto occlude the LAA and fluidically isolate the LAAfrom the left atrium; and
actuating a wall piercing arm of the device of the invention to generate a hole in the wall of the LAA, typically distal of the occlusion apparatus.

In one embodiment, the method of the invention employs a device of the invention in which the wall piercing arm is operatively coupled to the occlusion apparatus, the method comprising the steps of transluminally delivering the device to the left atrium of the heart and into a mouth of the LAA by means of a delivery catheter, deploying the occlusion apparatus within the LAA, and then deploying the wall piercing arm to generate the hole in the wall of the LAA. In one embodiment, deployment of the wall piercing arm comprises axially moving the wall piercing arm distally out of the through-conduit whereby the arm deploys to a wall piercing position.

In another embodiment, the method of the invention employs a device of the invention in which the wall piercing arm is a catheter that is separate from the occlusion apparatus and configured to pass through the through-conduit, the method comprising the steps of transluminally delivering the device to the left atrium of the heart and into a mouth of the LAA by means of a delivery catheter, deploying the occlusion apparatus within the LAA, detachment and retraction of the delivery catheter leaving the occlusion apparatus in-situ, transluminal delivery of the wall piercing catheter to the left atrium and through the through-conduit of the occlusion apparatus to a wall piercing position, and actuation of the catheter to generate a hole in the wall of the LAA.

In one embodiment, a guidewire is advanced through the wall piercing catheter into the epicardial space.

In one embodiment, the wall piercing catheter is retracted from the LAA, and a second sensing or treatment catheter is advanced transluminally over the guidewire and into the epicardial space. In one embodiment, the method includes a step of performing a treatment or sensing operation within the epicardial space using the treatment or sending catheter.

The invention also provides a system for occluding the LAA, generating a hole in a wall of the LAA when occluded, and then treating, stimulating or monitoring tissue, typically heart tissue, using a treatment or sending apparatus disposed in the epicardial space. In one embodiment, the invention provides a system comprising:
a device of the invention for providing transluminal access to the epicardial space via the left atrial appendage; and
a treatment or sensing catheter configured for (a) transluminal delivery through the through-conduit of the occlusion apparatus when deployed and into the epicardial space through the hole in the wall of the LAA or (b) apical delivery through the hole in the wall of the LAA and the through-conduit of the occlusion apparatus when deployed and into the left atrium.

The treatment or sensing catheter employed in the device and methods of the invention may be configured for performing a function selected from: ablation of tissue; epicardial surgery; endocardial surgery; minimally invasive Pericardiectomy; delivery of pacing leads; percutaneous valve repair or delivery; coronary artery modification; atrial or ventricle wall defect surgery; sensing a parameter of tissue optionally selected from temperature, impedance, oxygen levels and tissue mapping, stimulation of the oesophagus, the intrinsic cardiac nervous system to modify heart rate, atrial and ventricular refractoriness, contractility and blood flow, the left and right phrenic nerves, and the peri-oesophageal vagal nerves.

In one embodiment, the treatment or sensing catheter is configured to deliver radiofrequency energy (bipolar or monopolar), reversible or irreversible electroporation treatment, or nerve stimulation energy,

In one embodiment, the system may comprise a further treatment or sensing catheter configured for transluminal delivery to the left atrium.

In one embodiment, a distal end of each of the treatment or sensing catheters comprise a magnet or magnetisable element configured to provide magnetic attraction between the distal end of the catheters. The catheters may both be tissue ablation or sensing catheters configured to abut across a wall of the left atrium.

In one embodiment, the system comprises a hole-closure catheter configured to close the hole in the LAA wall after the treatment or sensing catheter has been removed. The catheter may be configured to deliver a bioglue, clip, suture, snare, or closure patch.

The invention also provides a system for treating or sensing a parameter of tissue for example heart tissue) comprising a first tissue treatment or sensing catheter and a second tissue treatment or sending catheter, in which each catheter is configured for delivery (generally transluminal delivery) to an endocardial or epicardial space, and in which a distal end of each of the treatment or sensing catheters comprise a magnet or magnetisable element configured to provide magnetic attraction between the distal end of the catheters.

In one embodiment, the catheters are treatment catheters configured to deliver radiofrequency energy (bipolar or monopolar), reversible or irreversible electroporation treatment, or nerve stimulation energy.

The invention also provides a method of for treating, or sensing a parameter of, a target tissue, especially heart tissue, that employs a system according to the invention, the method comprising the steps of:
providing transluminal access to the epicardial space via the left atrial appendage according to a method of the invention;
transluminally delivering a first treatment or sensing catheter into the epicardial space through the occlusion apparatus and hole in the wall of the LAA;
transluminally delivering a second treatment or sensing catheter into the chamber of the heart;
positioning the distal ends of the catheters on opposite sides of the target tissue; and
actuating the treatment or sensing catheters to perform a treatment or sensing function.

Typically, the target tissue is a wall of the heart, typically an external wall of the left atrium, although the method of the invention may be employed to treat or sense a parameter of another wall of the heart.

In another aspect, the invention provides a device for harvesting energy from the epicardial space via the left atrial appendage comprising:
an implantable occlusion apparatus configured for detachable attachment to an elongated catheter member and adjustment between a contracted orientation suitable for transluminal delivery and a deployed orientation configured to occlude the LAA (typically the ostium of the LAA), the implantable occlusion apparatus having a through-conduit and a cover disposed on a proximal end configured to fluidically isolate the LAA from the left atrium when the occlusion apparatus is deployed within the LAA, the cover comprising an opening to the through-conduit and a closure for the opening; and
an energy harvesting module operably coupled to the implantable occlusion apparatus and configured to extend through a hole in a wall of the LAA distal of the occlusion apparatus and into the epicardial space, and comprising one or more energy harvesting elements disposed within the epicardial space and configured to convert mechanical stress exerted on the element by the beating heart into electrical energy.

In one embodiment, the energy harvesting module comprises energy storage means coupled to the occlusion apparatus and configured to receive and store electrical energy form the energy harvesting elements.

In one embodiment, the occlusion apparatus comprises an electrically operated active element configured to treat or sense a parameter of tissue, in which the active element is operatively coupled to the energy harvesting module.

In one embodiment, the electrically operated active element is selected from a communication module, a pressure sensing module, a drug delivery device, a heart pacing device, an epicardial sensor, a peri-oesophageal sensor, a nerve sensing or stimulation device, a cardiac resynchronisation device, and a multi chamber pacing device.

In another aspect, the invention provides a device for providing access to the left atrium of the heart via the left atrial appendage comprising:
an implantable occlusion apparatus configured for detachable attachment to an elongated catheter member and adjustment between a contracted orientation suitable for transluminal delivery and a deployed orientation configured to occlude the LAA, the implantable occlusion apparatus having a through-conduit and a cover disposed on a proximal end configured to fluidically isolate the LAA from the left atrium when the occlusion apparatus is deployed within the LAA, the cover comprising an opening to the through-conduit and a closure for the opening; and
an introducer having a proximal part comprising a haemostasis valve, a distal part comprising an elongated conduit having a piercing tip and configured to extend into the LAA through the through-conduit in the occlusion apparatus and into the left atrium, and a through-lumen allowing access from the epicardial space to the left atrium through the introducer.

Other aspects and preferred embodiments of the invention are defined and described in the other claims set out below.

### Brief Description of the Figures

**Figure 1** illustrates a LAA occlusion device according to the invention for providing transluminal access to the epicardial space via the left atrial appendage, in which the wall piercing arms are attached to the occlusion apparatus.
**Figure 2** illustrates a LAA occlusion device according to the invention for providing transluminal access to the epicardial space via the left atrial appendage, in which the wall piercing arm is a catheter member configured to pass through the occlusion apparatus.
**Figure 3** illustrates a system for treating heart tissue using an epicardial catheter and endocardial catheter.
**Figure 4** illustrates a system according to the invention for treating a wall of the left atrium employing an endocardial catheter and an epicardial catheter.
**Figure 5** illustrates a LAA occlusion device according to the invention configured for harvesting energy from the epicardial space via the left atrial appendage.
**Figure 6** illustrates a LAA occlusion device of the invention for providing epicardial access to the left atrium.
**Figure 7** illustrates a device according to the invention in which the occlusion apparatus is deployed.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

As used herein, the term "treatment or sensing catheter" should be understood to include a catheter configured for performing a function selected from: ablation of tissue; epicardial surgery; endocardial surgery; delivery of pacing leads; percutaneous valve repair or delivery; coronary artery modification; atrial or ventricle wall defect surgery; and sensing a parameter of tissue optionally selected from pressure, temperature, impedance, oxygen levels and tissue mapping, communication with a remote device, drug delivery, heart pacing, epicardial sensing, nerve sensing or stimulation, a cardiac resynchronisation device, multi chamber pacing, ablation of tissue, epicardial surgery, endocardial surgery, delivery of pacing leads, percutaneous valve repair or delivery, coronary artery modification, atrial or ventricle wall defect surgery. The treatment or sensing catheter may be configured to deliver radiofrequency energy (bipolar or monopolar), reversible or irreversible electroporation treatment, nerve stimulation energy, thermal energy, cryogenic energy.

As used herein, the term "substance" refers to a fluid (liquid or gas), solid, semi-solid or gel. The substance may be a pharmaceutically active substance, for example a chemical or biological drug, for example an anti-microbial agent, an anti-inflammatory agent, a growth factor, an enzyme, a hormone, a protein, peptide, sugar, antibody (or antibody fragment), nucleic acid, nucleic acid construct, an imaging contrast agent, or a cooling liquid.

"Occlusion apparatus" means a body that is expansible from a contracted delivery configuration to an expanded deployed configuration. The apparatus may take many forms, for example a wireframe structure formed from a braided or meshed material. Examples of expandable wireframe structures suitable for transluminal delivery are known in the literature and described in, for example, WO01/87168, US6652548, US2004/219028, US6454775, US4909789, US5573530, WO2013/109756. Other forms of apparatus suitable for use with the present invention include plate or saucer shaped scaffolds, or inflatable balloons, or stents. In one embodiment, the apparatus is formed from a metal, for example a shape-memory metal such as nitinol. The apparatus may have any shape suitable for the purpose of the invention, for example discoid or spheroid. In one embodiment, the apparatus comprises a tissue ablation device. In one embodiment, the ablation device comprises an array of electrical components. In one embodiment, the array of electrical components is configured to deliver ablative energy in a specific pattern while mapping temperature. In one embodiment, the array of electrical components is configured for pacing the cardiac tissue for confirmation of ablation and disruption of chaotic signalling from the LAA. In one embodiment, a distal face of the apparatus comprises a covering configured to fluidically isolate the LAA from the left atrium, and optionally promote epithelial cell proliferation. In one embodiment, the apparatus comprises a stepped radial force stiffness profile from distal to proximal device. In one embodiment, the apparatus comprises a metal mesh cage scaffold. In one embodiment, a coupling between the apparatus and the catheter member is located distally to the left atrial facing side of the body. In one embodiment, the apparatus in a deployed configuration has a radial diameter at least 10% greater than the radial diameter of the left atrial appendage at a point of deployment. In one embodiment, the furthermost distal body is configured to be atraumatic to cardiac tissue. In one embodiment, the opening in the cover is configured to self-close on retraction of the delivery component (i.e. catheter member). In one embodiment, the apparatus comprises a braided mesh scaffold that in one embodiment is conducive to collagen infiltration on thermal energy delivery to promote increased anti migration resistance. In one embodiment, the array of electrodes generates an electrical map or profile of the ablation zone and the surrounding tissue electrical impedance measurements to characterise the electrical properties of the tissue, wherein the characterisation is optionally used as a measurement and confirmation of ablation effectiveness.

"Closure" or "Cover" typically means a layer disposed on the proximal side of the occlusion apparatus covering the opening into the recessed socket. It is intended to prevent blood flow past the occlusion apparatus into the LAA. It may be formed from a woven mesh material, and may include a re-closable closure, for example an overlapping flap of material or a polymeric valve, or it may comprise a pierceable cover. In some embodiment, the connecting hub is disposed in a recess between the cover and the concave proximal face of the radially expansible body.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

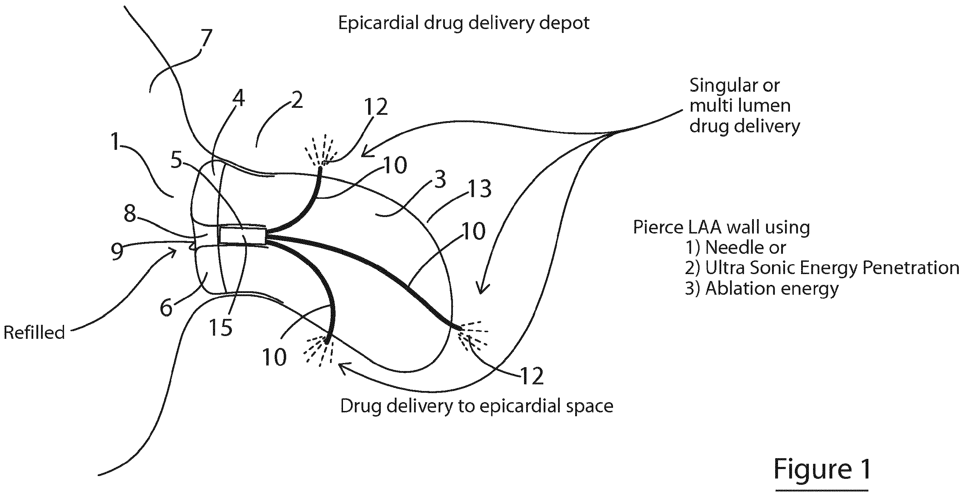

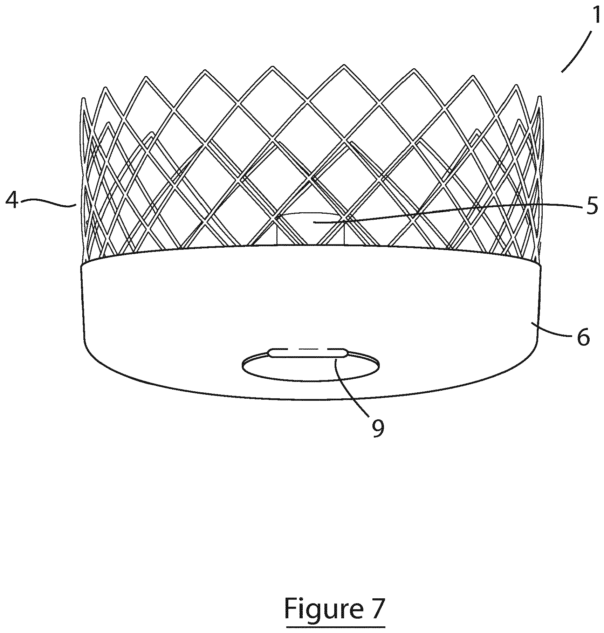

Referring to the drawings, and initially to Figure 1 and 7, there is illustrated a device according to the invention, indicated generally by the reference numeral 1, for transluminal access to the epicardial space 2 via the left atrial appendage (LAA) 3. The device 1 comprises an implantable occlusion apparatus 4 configured for detachable attachment to an elongated catheter member (not shown) and adjustment between a contracted orientation suitable for transluminal delivery (not shown) and a deployed orientation configured to occlude the LAA The implantable occlusion apparatus 4 has a through-conduit 5 and a cover 6 disposed on a proximal end configured to fluidically isolate the LAA from the left atrium 7 when the occlusion apparatus is deployed within the LAA. The cover 6 comprising an opening 8 to the through-conduit and a closure 9 for the opening.

Referring to Fig. 1, wall piercing arms 10, operably coupled to the implantable occlusion apparatus 4, are provided and are configured to generate holes 12 in a wall 13 of the LAA distal of the occlusion apparatus during use, to provide access to the epicardial space via the left atrial appendage. In this embodiment, the wall piercing arm 10 is attached to a module 15 housed within the conduit 5, and the arms are configured to splay outwardly when they are advanced distally beyond the through-conduit 5 of the occlusion apparatus and into engagement with the wall of the LAA. The arms may be made of a shape-memory material to allow deployment. In this embodiment, the arms 10 comprise a distal wall piercing needle (not shown) having a lumen with an outlet at a tip of the needle and in fluid communication with a substance reservoir disposed in the module 15. However, it will be appreciated that the arm may include other types of wall piercing means, including ablation electrodes and ultrasonic probes. The module 15 also includes a pump configured to pump the substance into the lumens in the arms, and a battery operably connected to the pump.

In use the device is delivered to the left atrium of the heart via a transluminal, trans-septal, approach, and advanced into the ostium of the LAA where it is deployed to occlude and fluidically isolate the LAA, before the delivery catheter is detached and retracted. The detachable module (including wall piercing arms) is then actuated to deploy the arms distal of the occlusion apparatus into contact with the wall of the LAA, where the needle at the tip of the arms punches a hole in the LAA. The module is then further actuated to start pumping a substance, for example a drug, through the needle into the epicardial space. Once the supply of substance has been extinguished, the reservoir in the module may be re-filled using a catheter, or the module may be retracted into a retraction catheter and removed from the both. The reservoir may be disposed on a proximal side of the cover, making it easier to access for re-filling. The method and device may be employed for delivery of drugs into the epicardial space, the oesophagus, the intrinsic cardiac nervous system to modify heart rate, atrial and ventricular refractoriness, contractility and blood flow, into the left and right phrenic nerves, into the peri-oesophageal vagal nerves, or for stimulation of the epicardial space, the oesophagus, the intrinsic cardiac nervous system (to modify heart rate, atrial and ventricular refractoriness, contractility and blood flow, the left or right phrenic nerves, and the peri-oesophageal vagal nerves. The wall piercing arms may include a stimulation module (for example a stimulation electrode).

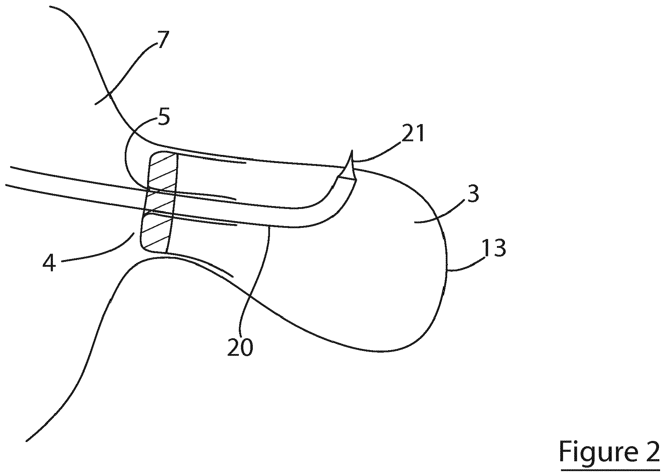

Referring to Figure 2, there is illustrated a device according to an alternative embodiment of the invention, in which parts similar to the those described with reference to the previous embodiment are assigned the same reference numerals. In this embodiment, the wall piercing arm is provided by a wall piercing catheter 20 having a piercing tip 21 configured for movement from a stowed, delivery, position within the catheter body and an exposed piercing position shown. The use of this embodiment involves delivering the occlusion apparatus to the LAA transluminally and trans-septally, deployment of the occlusion apparatus in the ostium of the LAA, detachment of the delivery catheter (not shown) from the occlusion apparatus and retraction from the body leaving the occlusion apparatus in-situ. The wall piercing catheter containing a guidewire is then advanced to the left atrium, through the conduit 5 in the occlusion apparatus, and is then steered into engagement with the wall of the LAA and actuated to expose the needle which pierces a hole in the wall of the LAA allowing the catheter to be advanced through the hole. The catheter is then retracted, leaving the guidewire in-situ spanning the wall of the LAA. A treatment or sensing catheter may then be advanced along the guidewire, through the occlusion apparatus and across the LAA wall into the epicardial space. This embodiment employs a steerable catheter, the details of which would be known to a person skilled in the art.

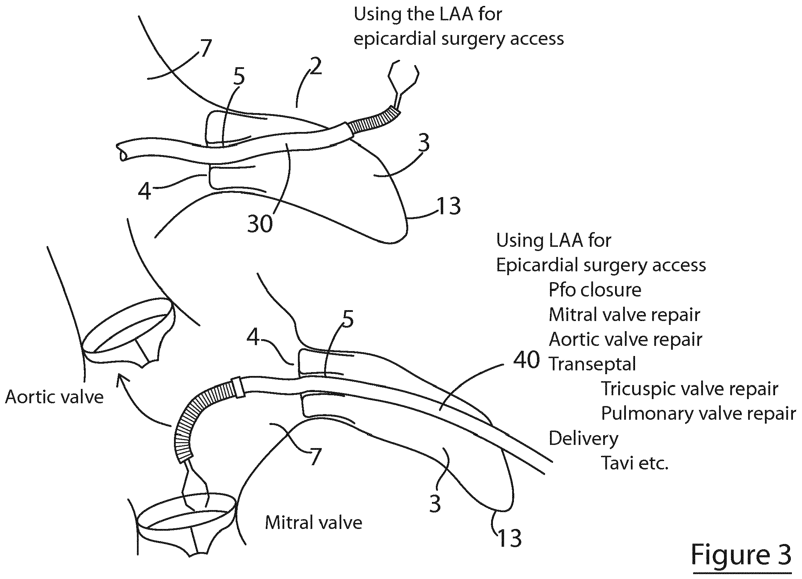

Figure 3A illustrates the use of a device of the invention for performing surgery in the epicardial space, in which the LAA is occluded and a hole generated in the wall of the LAA using the device and method of Fig. 2, and a catheter member 30 configured for performing a surgical procedure is advanced along the guidewire and into the epicardial space 2.

Fig. 3B illustrates the use of a device of the invention for providing access into a chamber of the heart via the epicardial space 2 and LAA. In this embodiment, a device according to the invention is employed to occlude the LAA and a wall piercing catheter is passed through the occlusion device 4 and actuated to generate a hole in the wall of the LAA, leaving a guidewire in-situ spanning the wall of the LAA. A snare disposed on the epicardial side may them be employed to snare the end of the guidewire, and draw it out of the body apically through the ribs. A surgical catheter 40 is then advanced apically over the guidewire, into the epicardial space, through the hole in the LAA wall, through the conduit in the occlusion apparatus, and into the left atrium, where the catheter can perform surgery on a heart valve, for example the aortic, pulmonary or tricuspid valve. It will be appreciated that the catheter may also be configured for delivery and implantation of a valve, or for other endocardial surgical procedures.

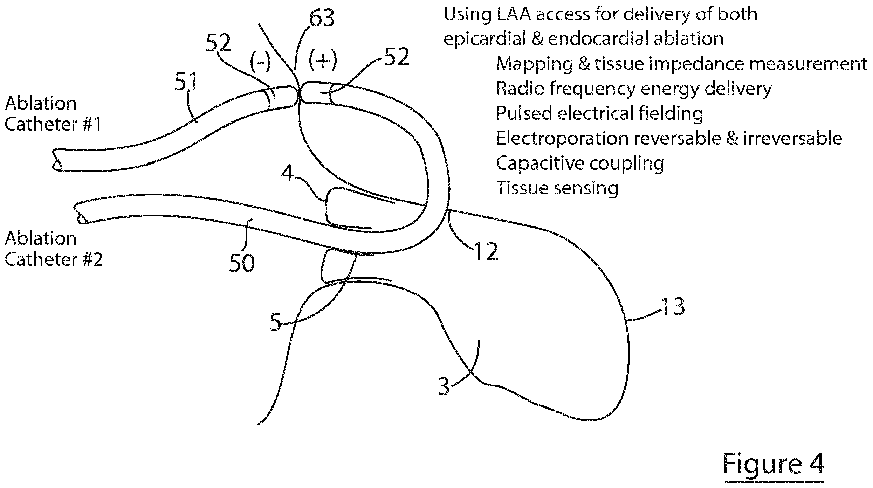

Figure 4 illustrates the use of a device of the invention for treating tissue, in which the LAA is occluded and a hole generated in the wall of the LAA using the device and method of Fig. 2. In this embodiment, a first tissue treatment catheter 50 is then delivered transluminally and trans-septally to the left atrium along a guidewire, through the conduit 5 in the occlusion apparatus 4 and through the hole 12 in the LAA into the epicardial space 2, where the catheter is remotely steered towards a target site on an external wall of the left atrium. A second treatment catheter 51 is then advanced to the left atrium, and steered into a position abutting the first catheter 50 across the target tissue. Correct positioning of the catheters is achieved by magnets 52 disposed on the tips of the catheters. Once in position, the catheters are actuated to perform a tissue treatment procedure on the target tissue. The treatment may be achieved by delivering RF energy, ultrasonic energy, pulsed electrical fielding, laser energy, microwave energy, electroporation, capacitive coupling. In another embodiment, the catheters may be configured to for sending a parameter of the tissue, for example tissue mapping. In another embodiment catheter 50 may have a distal balloon configured to anchor the distal part of the catheter within the epicardial space for optimal energy delivery.

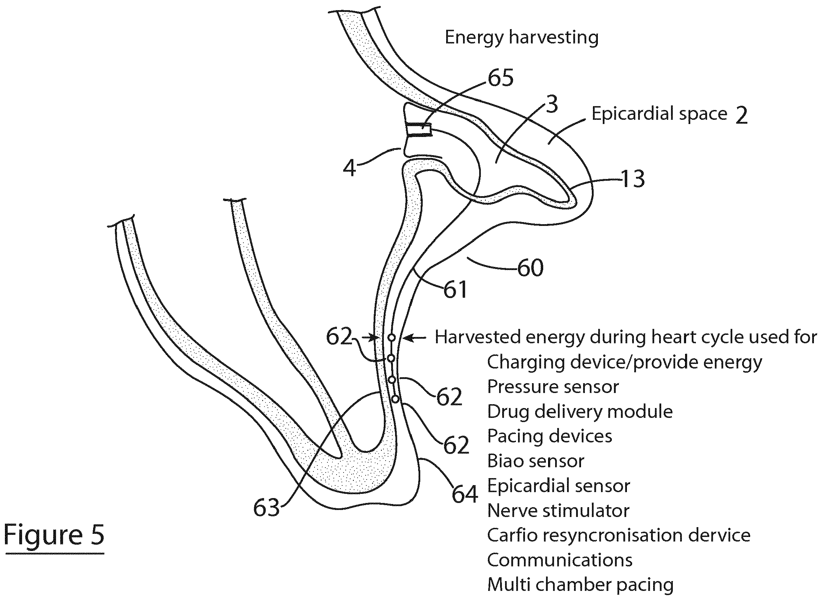

Referring to Figure 5, a LAA occlusion device according to an alternative embodiment of the invention is described, in which parts similar to those identified with reference to previous embodiment are assigned the same reference numerals. In this embodiment, the occlusion apparatus 4 comprises an energy harvesting module 60 operably coupled to the implantable occlusion apparatus and configured to extend through a hole in a wall of the LAA distal of the occlusion apparatus and into the epicardial space 2. The module comprises an electrical lead 61 having a number of piezoelectric energy harvesters 62 disposed on a distal end thereof, within the epicardial space 2 between the external wall of the left atrium 63 and the pericardium 64, configured to convert mechanical stress exerted on the element by the beating heart into electrical energy. The energy harvesting module 60 comprises energy storage means 65 coupled to the occlusion apparatus and configured to receive and store electrical energy form the energy harvesting elements. Generally, the occlusion apparatus comprises an electrically operated active element configured to treat or sense a parameter of tissue, in which the active element is operatively coupled to the energy harvesting module. The electrically operated active element may be selected from a communication module, a pressure sensing module, a drug delivery device, a heart pacing device, an epicardial sensor, a nerve sensing or stimulation device, a cardiac resynchronisation device, and a multi chamber pacing device.

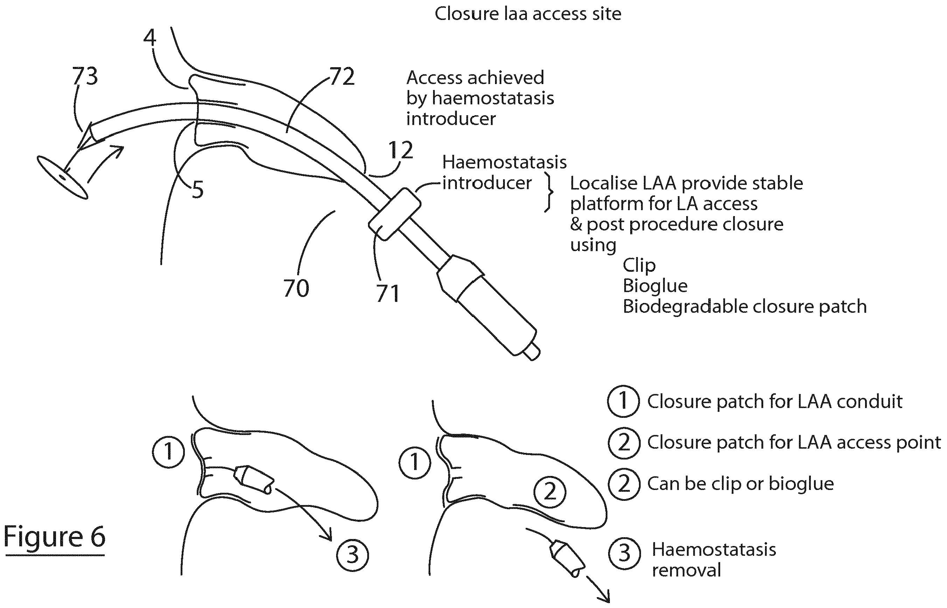

Referring to Figure 6, a device for providing access to the left atrium of the heart via the left atrial appendage is described, in which parts similar to those identified with reference to previous embodiment are assigned the same reference numerals. In this embodiment, the device comprises an introducer 70 having a proximal part comprising a haemostasis valve 71, a distal part comprising an elongated conduit 72 having a piercing tip 73 and configured to extend into the LAA through the hole 12 in the all of the LAA and through-conduit 5 in the occlusion apparatus 4 and into the left atrium, and a through-lumen allowing access from the epicardial space 2 to the left atrium through the introducer. In use, the LAA is first occluded using an occlusion apparatus 5 which is delivered to the LAA transluminally as described with reference to Fig. 2. The introducer 70 is then delivered into the epicardial space 2 apically, and pushed through the wall of the occluded LAA and advanced through the LAA and through the conduit 5 in the occlusion apparatus 4 and into the left atrium. The introducer then serves as a stable access point for delivering apparatus into the heart through the LAA. When the introducer is removed, the hole is the LAA can be closed using a clip, bioglue, rivet, fastener, or biodegradable closure patch as illustrated in Fig. 6.

It will be appreciated that the occlusion apparatus may be removed from the LAA after the treatment or sensing procedure has been completed, and the hole in the wall of the LAA has been closed. A retraction catheter may be employed for this purpose. Various means are envisaged for closing the hole in the wall of the LAA including use of bio-glues, clips, fasteners, and patches. In one embodiment, the distal part of the LAA can be clipped to the epicardium or heart tissue to seal. In one embodiment, a snare can be employed to close the hole from the inside out.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

**1.** A device (1) for providing transluminal access to the epicardial space (2) via the left atrial appendage (LAA) (3) comprising:
an implantable occlusion apparatus (4) configured for detachable attachment to an elongated catheter member and adjustment between a contracted orientation suitable for transluminal delivery and a deployed orientation configured to occlude the LAA, the implantable occlusion apparatus having a through-conduit (5) and a cover (6) disposed on a proximal end configured to fluidically isolate the LAA from the left atrium (7) when the occlusion apparatus is deployed within the LAA, the cover comprising an opening (8) to the through-conduit and a closure (9) for the opening; and
a wall piercing arm (10, 20) configured to cooperate with the implantable occlusion apparatus and configured to generate a hole (12) in a wall of the LAA distal of the occlusion apparatus during use to provide access to the epicardial space (2) via the LAA.

**2.** A device as claimed in Claim 1 in which the wall piercing arm comprises a distal wall piercing head selected from a wall-piercing needle (21), ultra-sonic probe, or tissue ablation electrode.

**3.** A device as claimed in Claim 1 or 2 in which the wall piercing arm (10) is attached to the implantable occlusion apparatus (4).

**4.** A device as claimed in any of Claims 1 to 3 in which the wall piercing arm (10) is attached to a control module (15) disposed within the through-conduit (5).

**5.** A device as claimed in any preceding Claim, in which the wall piercing arm (10) comprises a substance delivery lumen configured to deliver a substance into the epicardial space (2).

**6.** A device as claimed in Claim 4 and 5 in which control module (15) comprises a reservoir for substance that is configured for fluidic connection to the lumen of the wall piercing arm (10).

**7.** A device as claimed in any of Claims 1 to 4, including a plurality of wall piercing arms (10).

**8.** A device as claimed in any preceding Claim, in which the wall piercing arm (10) is axially adjustable relative to the implantable occlusion apparatus (4).

**9.** A device as claimed in Claim 1 or 2, in which the wall piercing arm comprises an elongated wall piercing catheter (20) configured for (a) insertion from the left atrium into the LAA through the through-conduit (5) of the deployed occlusion apparatus (4) and/or (b) insertion from the epicardial space into the left atrium through the hole (12) in the LAA wall and the through-conduit (5) of the deployed occlusion apparatus (4).

**10.** A device as claimed in Claim 7, in which the wall piercing catheter (20) is configured for guided movement.

**11.** A device as claimed in Claim 7 or 8, in which the wall piercing catheter (20) has a distal end configured to treat or stimulate tissue, or sense a parameter of tissue.

**12.** A device as claimed in any preceding Claim, including a delivery catheter for the occlusion apparatus (4) configured for detachable attachment to the proximal end of the occlusion apparatus.

**12.** A system for treating, stimulating or monitoring tissue comprising:
a device of any of Claims 9 to 11 for providing transluminal access to the epicardial space via the left atrial appendage; and
a treatment or sensing catheter (30, 50) configured for transluminal delivery through the through-conduit (5) of the occlusion apparatus (4) when deployed and into the epicardial space through the hole in the wall of the LAA.

**13.** A system according to Claim 12, in which the treatment or sensing catheter (30, 50) is configured for performing a function selected from: ablation of tissue; epicardial surgery; endocardial surgery; delivery of pacing leads; percutaneous valve repair or delivery; coronary artery modification; atrial or ventricle wall defect surgery; and sensing a parameter of tissue optionally selected from temperature, impedance, oxygen levels and tissue mapping, or in which the treatment or sensing catheter is optionally configured to deliver radiofrequency energy (bipolar or monopolar), reversible or irreversible electroporation treatment, or nerve stimulation energy,

**14.** A system according to Claim 13, including a further treatment or sensing catheter (51) configured for transluminal delivery to the left atrium, in which a distal end of each of the treatment or sensing catheters comprise a magnet or magnetisable element (52) configured to provide magnetic attraction between the distal end of the catheters.

**15.** A device for harvesting energy from the epicardial space (2) via the left atrial appendage (3) comprising:
an implantable occlusion apparatus (4) configured for detachable attachment to an elongated catheter member and adjustment between a contracted orientation suitable for transluminal delivery and a deployed orientation configured to occlude the LAA, the implantable occlusion apparatus having a through-conduit (5) and a cover (6) disposed on a proximal end configured to fluidically isolate the LAA from the left atrium when the occlusion apparatus is deployed within the LAA, the cover comprising an opening (8) to the through-conduit and a closure (9) for the opening; and
an energy harvesting module (6) operably coupled to the implantable occlusion apparatus (4) and configured to extend through a hole (12) in a wall of the LAA distal of the occlusion apparatus and into the epicardial space (2), and comprising one or more energy harvesting elements (62) disposed within the epicardial space and configured to convert mechanical stress exerted on the element by the beating heart into electrical energy.
